(19) Europäisches Patentamt
European Patent Office
Office européen des brevets

(11) EP 0 943 680 A1

# (12) EUROPEAN PATENT APPLICATION

(43) Date of publication:
**22.09.1999 Bulletin 1999/38**

(51) Int. Cl.[6]: **C12N 9/42**, A61K 48/00

(21) Application number: **98104756.6**

(22) Date of filing: **17.03.1998**

(84) Designated Contracting States:
**AT BE CH DE DK ES FI FR GB GR IE IT LI LU MC NL PT SE**
Designated Extension States:
**AL LT LV MK RO SI**

(71) Applicant:
**Boehringer Ingelheim International GmbH**
**55216 Ingelheim (DE)**

(72) Inventors:
- **Izquierdo, Marta, Prof.**
  **28761 Madrid (ES)**
- **Cortés, Maria Luisa, Dr.**
  **28224 Madrid (ES)**

## (54) Suicide gene therapy system for the treatment of brain tumours

(57) In a suicide gene therapy system, a DNA molecule encoding a glycosidase is applied with a non-toxic cyanogenic glycoside substrate, which upon degradation by the glycosidase yields hydrogen cyanide, for the treatment of brain tumours in animals or humans. The DNA preferably encodes linamarase, the substrate is preferably linamarin. The two interacting components are administered separately.

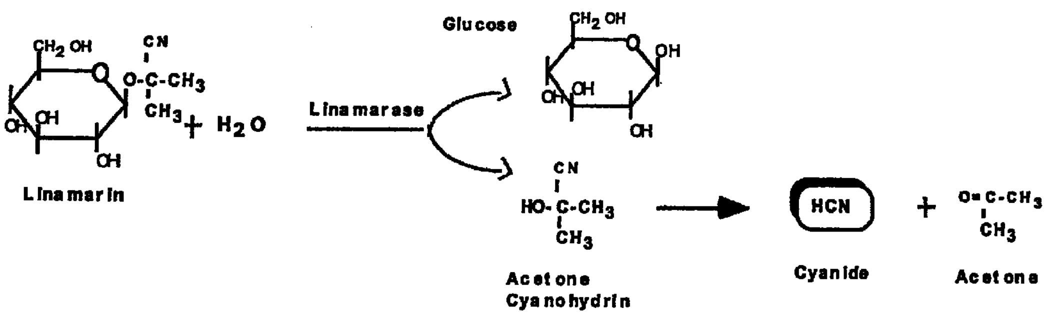


Linamarin

Glucose

Acetone Cyanohydrin

Cyanide

Acetone

Fig. 1



Printed by Xerox (UK) Business Services
2.16.7/3.6

## Description

The invention relates to the field of gene therapy for brain tumours.

Glioblastoma is one of the most frequent (1 per 50,000 people per year) and deadly (mortality close to 100%) brain tumours in humans. In order to find a cure for this disease, a number of strategies have been developed in the past. One of the most promising strategies was to sensitize tumour cells to specific prodrugs. Among these strategies, one of the most successful systems to date is the herpes simplex thymidine kinase (Hs*tk*)/ganciclovir (Culver et al., 1992; Ram et al., 1993; Izquierdo et al., 1995). HsTK phosphorylates nucleoside analogs (such as ganciclovir or acyclovir) that normal eukaryotic cells fail to phosphorylate. The incorporation of these analogs into the replicating DNA blocks the process, thereby killing the cell. Malignant brain tumours (glioblastoma) have been cured in the rat animal model provided the volume of the tumour does not exceed 100-150 $mm^3$ in size (Izquierdo et al., 1997). When the same treatment is applied to humans, complete remission of the tumour is not achieved, although there is, in some cases, a measurable size reduction probably responsible for a considerable increase in the expected survival time of these patients (Culver et al., 1994; Izquierdo et al., 1996). In a similar way, tumour cells may be sensitized to cytosine arabinoside (ara-C) or 2'-2'difluorodeoxycytidine (gemcitabine) by delivery to the neoplastic cell of the deoxycytidine kinase gene enabling the prodrugs to be phosphorylated and thus block replication. The cytosine deaminase gene, as a further example, is capable of making cells sensitive to the prodrug 5-fluorocytosine.

To date, only four prodrug activating systems have been used in transgene systems for brain tumours: the above-mentioned herpes thymidine kinase for the activation of ganciclovir and the *E. coli* cytosine deaminase for 5-fluorocytosine, the human cytochrome P450 2B1 for cyclophosphamide, and the *E. coli* purine nucleoside phosphorylase for activation of 6-methyl-purine-2'-deoxyribonucleoside.

It has already been suggested by Deonarain, 1994, to explore the enzyme linamarase, which converts amygdalin to cyanide, for the treatment of hepatoma, melanoma, pancreatic and breast cancer.

It was the object of the present invention to provide an alternative killer-suicide gene therapy system for the treatment of brain tumours, in particular of the malignant type such as glioblastoma.

To solve the problem underlying the object of the invention, an alternative suicide gene system for the preparation of a medicament for the treatment of brain tumours was developed.

The present invention is directed to the use of a DNA molecule encoding a glycosidase in combination with a non-toxic cyanogenic glycoside which upon degradation by the glycosidase yields hydrogen cyanide, for the preparation of a medicament for the treatment of brain tumours.

Glycosidases (O-Glycosyl hydrolases) are a widespread group of enzymes which hydrolyse the glycosidic bond between two or more carbohydrates or between a carbohydrate and a non-carbohydrate moiety. The catabolism of cynogenic glycosides is initiated by cleavage of the carbohydrate moiety by a β-glucosidase, which yields the corresponding alpha-hydroxynitrile. The hydroxynitrile decomposes by producing hydrogen cyanide and an aldehyde ketone.

In a preferred embodiment of the invention, the glycosidase is a glucosidase, in particular a β-glucosidase, and the substrate is a cyanogenic glucoside.

In an even more preferred embodiment of the invention, the enzyme β-glucosidase is linamarase and the cyanogenic glucoside is linamarin. The linamarase hydrolyzes the innocuous substrate linamarin (2-0H-isobutyronitrile-β-D-glucopyranoside) to acetone cyanohydrin and glucose (Mkpong et al., 1990; Hughes et al., 1992) (Fig. 1). Acetone cyanohydrin is unstable at pH above 6 and spontaneously decomposes to acetone and HCN (Selmar et al., 1987a, b). The cyanogenic glucoside, linamarin, is not hydrolyzed by most mammalian tissues, therefore in the absence of linamarase, once absorbed, it is excreted unchanged in the urine (Frakes and Sharma, 1986; Hernandez et al., 1995).

The β-glucosidase , e.g. linamarase, may be derived from different plants, e.g. cassava, flax, hevea, phaseolus, white clover, etc. (Kakes, 1985; Fieldes and Gerhardt; 1994; Frehner and Conn, 1987; Hughes and Dunn, 1982).

Alternatively to linamarase and linamarin, other glycosidases and corresponding substrates may be employed. Depending on the sugar moiety and the glycosidic bond, the enzyme may be selected, inter alia, from monosaccharidases and disaccharidases (e.g. galactosidases, lactases, fucosidases). The enzymes may be derived from plants or microorganisms. To obtain the DNA encoding an enzyme whose sequence has not yet been identified, methods based on standard protocols in molecular biology (Sambrook et al., 1989) can be employed. An example for such a method was used for determining the nucleotide and amino acid sequence of the linamarase from white clover (Oxtoby et al., 1991).

Apart from naturally occuring enzymes, or DNAs encoding them, respectively, modified enzyme molecules, or DNAs encoding them, respectively, may be used. Such molecules may be truncated or modified, as long as they hydrolize the cyanogenic carbohydrate substrate, such that the toxic agent HCN is released. Whether a modified molecule is suitable for application in the present invention, can be easily verified in preliminary enzymatic assays in which the substrate is incubated with the enzyme and the release of HCN is detected.

Besides linamarin, the substrate may be selected from other naturally occuring cyanogenic glycosides, e.g. amygdalin, sambunigrin, prunasin, holocalin, zierin, lotaustralin, taxiphyllin, vicianin, dhurrin (Gmelin et al., 1973;

Jensen et al., 1973; Stevens et al., 1968; Nartey, 1968).

Apart from naturally occuring cyanogenic glycoside substrates, synthetic cyanogenic glycosides may be designed to be used as substrate for the enzyme.

Examples for synthetic substrates may be monosaccharides based on those with the general formula

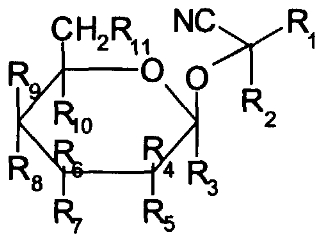

wherein $R_1$ and $R_2$, which may be different from one another or the same, denote branched or unbranched $C_1$-$C_4$-alkyl; $R_3$, $R_4$, $R_5$, $R_6$, $R_7$, $R_8$, $R_9$, $R_{10}$ and $R_{11}$, which may the same or different fom one another, denote hydrogen, hydroxy $C_1$-$C_4$-alkyl or $C_1$-$C_4$-alkoxy.

Similarly the expert may design corresponding disaccharide derivatives, for example derivatives of cellobiose, maltose, lactose, saccharose, gentobiose, trehalose or turanose.

In order for a substrate to be suitable for the present invention, it must be non-toxic and amenable to decomposition by a glycosidase to yield hydrogen cyanide. The person skilled in the art may test the suitability of an enzyme/substrate combination in routine enzyme assays, in which the substrate is incubated with the enzyme under the appropriate conditions and the production of hydrogen cyanide is determined, as described, inter alia, by Selmar et al., 1987; Itoh-Nashida et al., 1987; Mao and Anderson, 1967; Stevens et al., 1968.

The invention makes, for the first time, use of a DNA molecule, whose product action provokes release of hydrogen cyanide, for the treatment of brain tumours, in particular malignant brain tumours.

In the following, for simplicity, the term "linamarase gene" is used for the DNA molecule encoding a glycosidase.

The invention may be applied for the treatment for tumours like glioblastoma, meduloepitelioma, meduloblastoma, neuroblastoma, Germinoma, embryonic carcinoma, malignant astrocytoma, astroblastoma, ependimoma, oligodendroglioma, plexal carcinoma, neuroepitelioma, pineoblastoma, ependimoblastoma, primitive neuroectodermic tumour, malignant meningioma, condrosarcoma, meningeal sarcomatosa, malignant melanoma, malignant schwanoma.

The medicament of the present invention represents a combined preparation of two ingredients, i.e. the linamarase gene and the substrate linamarin, which are administered separately. The two components interact directly and form a functional unity.

In the present invention, the linamarase gene may be used in combination with any vector system that is suitable for delivering genes into brain tumour cells.

In a preferred embodiment, the linamarase gene is carried by a viral vector.

In a particularly preferred embodiment, the vector is a retroviral vector. Retroviruses only effectively infect proliferating cells (although they could enter quiescent ones) because they require cell division for integration and gene expression. This property makes retrovirus the most convenient choice vectors for the treatment of rapidly dividing cells in malignancies arising within a postmitotic tissue, in an anatomical compartment (the brain) that is partially separated from the rest of the body by the hematoencephalic barrier. When compared to other types of viral vectors, retroviral vectors are more selective for transgene delivery to brain tumour cells, and the transgene is inherited by all progeny cells of the infected cells.

The vector must fulfil the general requirement to be defective and therefore unable to give infective progeny. It has two long terminal repeats (LTR) acting as potent promoters and a selection gene, e.g. the puromycin resistance gene (puromycin N-acetyl transferase), besides the therapeutically active linamarase gene. A great variety of retroviral vectors have been designed for gene therapy applications and are available to the person skilled in the art. Examples for suitable vectors that may be used in the present invention and methods for constructing such vectors have been reviewed by Vile and Russel, 1995, Braas et al., 1996. Most retroviral vectors that have been designed for gene therapy are based on Moloney-Murine Leukemia Virus (Mo-MLV). A survey of retroviral vectors and the requirements upon them that need to be fulfilled in order to make them suitable for use in brain tumour gene therapy is given in the article Kramm et al., 1995, the disclosure of which, including the relevant cited references, is herewith expressly incorporated by reference herein.

The retroviral vector may be applied as such, i.e. as a constituent of the pharmaceutical composition, together

with an agent such as protamine or polybrene to facilitate viral infection, in a suitable physiological buffer, usually a saline buffer. The viral titer should not be lower than $10^6$ colony forming units per ml. The preferred route of administration is intratumoral injection of the virus and infusion for the substrate.

In order to counteract the limitations of the use of retroviral vectors *per se,* i.e. direct application of the virus by intratumoral injection, which are due to the relatively low titers and instability of infectious particles, the retroviral vectors are preferably applied by intratumoral grafting of packaging cells (producer cells) that have been infected with the vector and allowed to grow. The packaging cells are injected as a suspension in saline buffer and can be of human or murine origin, preferably human, e.g. cells of the human fibrosarcoma cell line FLYA13 (Cosset et al., 1995). An example for a mouse cell line is ΨCRIP (Danos and Mulligan, 1988). Human cells are preferred because they are not rejected as easily as murine producer cells. The cells should give titers not inferior to $10^6$ colony forming units per ml, the cell number injected is approximately $10^9$ or above.

In another embodiment, the viral vector is an adenoviral vector. Due to the natural tropism of adenovirus, its accessibility to easy manipulation and the high viral titers that can be obtained, adenoviral vectors have found increased interest for cancer gene therapy as an alternative to retroviral vectors, the use of which is limited due to their requirement for active cell division and by the viral titers that can be obtained. A great variety of adenoviral vectors is available for use in the present invention, a review on recent vector developments that take into account the structural, immunological and targeting problems posed by gene therapy applications, is given by Kovesdi et al., 1997. Strategies for cancer therapy using adenoviral vectors were also evaluated by Descamps et al., 1996; specifically, adenoviral vectors have been shown to be useful for applying the HSV-tk/ganciclovir suicide system in the treatment of glioma (Chen et al., 1994; Bonnekoh et al., 1995). Adenovirus-mediated gene transfer to the brain has been further discussed by Kramm et al., 1995, and Yung, 1994; and methodologically assessed by Peltékian et al., 1997. The vectors disclosed in the cited references can be used for inserting the linamarase gene according to the present invention.

Since adenoviruses can be produced with higher titer numbers, they can be applied as such, i.e. there is no requirement for implanting producer cells.

According to a further embodiment of the invention, adenoviral/retroviral vector chimeras may be used that combine the advantageous features of both adenoviral and retroviral vectors and have been recently shown to provide a novel strategy to achieve high-efficiency stable transfection *in vivo* (Bilbao et al., 1997).

In another embodiment of the invention, the viral vector is a Herpes simplex virus (HSV) vector. Methods for constructing HSV vectors that may be used for inserting the linamarase gene and delivering the gene to the tumour cells, have been described by Latchman, 1994; the use of HSV vectors for brain tumours has been disclosed by Kramm et al., 1995; and Yung, 1994.

Regarding the other components of the pharmaceutical composition that contains a viral vector carrying the linamarase gene, they are essentially the same as for the retroviral vector compositions. The pharmaceutical composition may contain a polycationic agent that enhances viral binding to the host cells in vivo, such as polybrene (hexadimethrine bromide) or protamine, which may be present in a concentration of approximately 4-8 μg/ml. When applying such agents, the risk of possible side effects, e.g. hemorrhage in case of protamine, has to be weighed against the benefit of an increase of gene transfer efficacy achieved by the addition of that agent.

Apart from viral vector systems, non-viral gene delivery systems may be used in the present invention to deliver a plasmid that carries the linamarase gene to the brain tumour cells. By using non-viral systems, some of the disadvantages of viral vectors, e.g. design constraints and safety issues, can be avoided. A non-viral system that may be used in the present invention is based on receptor-mediated endocytosis. Such a system is disclosed in WO 93/0783 which is based on complexes of plasmid DNA and polycations that are conjugated with cell surface binding ligands and is equipped with an endosomolytic function, such as an inactivated adenovirus or a membrane active peptide. For the present invention, the cell surface binding ligand that serves as targeting function may be selected from brain cell binding ligands, e.g. glioma-specific antibodies.

Another group of non-viral gene transfer systems that can be used in the present invention is based on lipophilic gene delivery vehicles. Examples for such delivery vehicles are liposomes which have been suggested for various gene therapy applications (Felgner et al., 1994; San et al., 1993). The plasmid carrying the linamarase gene can be encapsulated into liposomes according to known methods (Hug and Sleight, 1991). Other examples are cationic lipids such as lipofectin (Felgner et al., 1987). To increase the uptake of non-viral delivery vehicles and to enhance linamarase gene expression, several routes of administration that take into account the specific requirements posed by the necessity to open the blood-tumour barrier in the brain have been suggested for the application of drugs to brain tumours may be applied in the present invention as an accompanying measure. Such routes have been reviewed by Kramm et al., 1995; an example is the application of bradykinin or bradykinin analogues. A further example to improve uptake of the gene by brain tumor cells is the conjugation of targeting molecules to the delivery vehicle, e.g. coupling liposomes with a glioma-specific antibody, as suggested by Yoshida and Mizzuno, 1994. Targeting molecules may also be monoclonal antibodies to proteins that are more abundant in gliomas, such as the fibrillary acidic protein (Abaza et al., 1997).

An alternative route for applying the linamarase plasmid is the transfer of naked DNA. In principal, this transfer method would, for several economical and technical reasons, be an ideal system, furthermore it is advantageous due to the low antigenicity of naked DNA. The disadvantage of the low efficiency of this gene transfer route has lately been overcome by a method that combines plasmid injection followed by *in vivo* electroporation of the tumour tissue. My means of electroporation, cell membranes are permealized, thus markedly enhancing drug and/or gene transfer (Nishi et al., 1996).

As for the viral systems, the preferred route of administration for the above-mentioned non-viral gene delivery systems is intratumoral injection.

Administration of the prodrug, e.g. linamarin, is preferably local, although it may also be peritoneal or intravenous. The preference for local administration is based on the estimation and experimental confirmation in other systems (tk/ganciclovir or tk/5-2-bromovinyl-2'-deoxyuridine) that the amount of prodrug needed to eradicate 1 $mm^3$ of tumour is one thousand times lower if the prodrug is administered locally than intraperitoneally, which substantially decreases the cost of the treatment and reduces possible adverse effects on other tissues.

Local administration may be achieved by infusion with a cannula from the tumour cavitiy to the outside, connected for several hours a day with a continuos infusion pump, that will deliver approximately 0.2 ml/hour. The treatment lasts for about a week. The total dose is approximately 50 mg of linamarin per day. This is equivalent to 5 mg of cyanide per day, which is far from the toxic doses of 50-60 mg. The prodrug is preferably administered in a physiological salt solution, e.g. PBS (phosphate buffered saline).

In general, 50mg of the prodrug is the minimum dose per day that is administered for treatment of a tumour. This dosis, which is far below the toxic concentration in terms of the released cyanide, may be increased in order to eradicate larger tumours.

Depending on the specific requirements of the treatment, i.e. the individual patient, the size and localization of the tumour, the number of tumours, etc., the mode of application of the substrate may be continuous, which is the preferred mode of application, or sequential in several doses per day, or which may, in some cases, be one single dose. Consequently, the substrate concentration may vary. In case of continuous administration, the minimum substrate concentration is 10 mg/ml. If the substrate is administered in one single or several doses, the concentration may be increased accordingly to approximately 30mg/ml.

The substrate is administered after a time sufficient for the linamarase gene to be expressed in the tumour cells. If, according to the preferred embodiment of the invention, the linamarase gene is administered by means of a retroviral vector, this period of time is determined by the rate of infection of the tumour cells by the virus. Depending on the accessibility of the tumour cells, this time is usually several days to approximately a week.

In a preferred embodiment, the invention comprises a kit, wherein a first container contains the preparation with the DNA encoding the glycosidase, preferably the linamarase, and a second container contains the preparation with the substrate, preferably linamarin. Both preparations are in pharmaceutically acceptable formulations, e.g.physiological salt solutions. In a preferred embodiment, the first container contains retroviral vectors carrying the linamarase gene, with a titer of $\geq 10^6$ colony forming units. In a preferred embodiment, the producer cells, infected with the retroviral vectors, are provided in deep frozen form. The number of cells contained in the preparation may be the number of cells ready for application, i.e. $10^9$ cells or more, alternatively, a stock preparation of cells is provided to be grown in the appropriate medium, which may be provided in a further container, to obtain the number of cells required for the specific application.

The second container contains the linamarin. In an preferred embodiment, the linamarin is present in solid form in the total dosis to be applied, and the buffer is provided separately. In this embodiment, the substrate solution may be prepared prior to administration in a suitable concentration according to the administration route.

It has been shown in the experiments of the present invention that neoplastic retroviral cells that express the linamarase gene have, upon introduction into tumour cells, the potential of causing a large bystander effect among neighbouring cells. The data obtained suggest a substantial killing effect that would counteract the poor estimates (1-10%) for *in vivo* retroviral infection of solid tumours. The experiments conducted in the present invention demonstrate that transduction of mouse or rat cells by retroviral vectors expressing the cassava linamarase gene, *cas 5* (Hughes et al., 1992), drastically increases sensitivity of these cells to the cytotoxic effects of linamarin *in vitro* and *in vivo*. Neighbouring cells can also be affected by the cyanide released, thereby amplifying the killing effect.

In the experiments of the present invention a therapeutic retrovirus was engineered by inserting the linamarase gene (*cas 5*) into the Moloney murine leukaemia based vector pBabe*puro* (Morgenstern and Land, 1990). The resulting recombinant retroviral plasmid carries the *cas 5* gene expressed under the control of the 5' LTR (long terminal repeat) promoter and the selection gene *pac* (puromycin N-acetyl transferase) under the SV40 promoter.

Amphotropic retroviral packaging cells ΨCRIP were grown in increasing amounts of linamarin to determine the sensitivity of these cells to the compound. Values up to 2 mg/ml of linamarin did not substantially decrease cell survival values. No cyanide was detected in any type of cell line tested (C6, ΨCRIP and 3T3) even after treatment with very high concentrations of linamarin, showing the stability of the substrate and the absence of linamarase activity in these mammalian cells.

The ecotropic murine packaging cell line ΨCRE was transfected by the retroviral plasmid vector *pLlinSp* and supernatants from ΨCRE lawns, were collected to infect ΨCRIP producing individual clones. Some clones showed a marked toxicity to the presence of linamarin, with estimated concentrations that resulted in 50% cytotoxicity ($IC_{50}$), of 7-60 μg/ml (Fig. 3a). Toxicity is due to cyanide production as measured by the Lambert method (Lambert et al 1975).

In a similar way, rat glioblastoma C6 were infected with ΨCRE*lin*. The C6*lin* clones gave in general $IC_{50}$ 50 times higher than ΨCRIP individual clones. Cells of the packaging cell line FlyA13 (human fibrosarcoma) have an intermedian $IC_{50}$ value of 195 μg/ml at pH 7.4 that drops to 77 μg/ml at pH 6.6 (Fig.3c). Other cell lines whose $IC_{50}$ were estimated showed values loser to C6 than to ΨCRIP (Table 1).
The extraordinarily low $IC_{50}$ value estimated for ΨCRIP could be related to a very high linamarin permeability of these particular cells. Upon incubation with linamarin, cell extracts from C6*lin* produced even higher amounts of cyanide than ΨCRIP*lin* suggesting a very efficient expression of the gene in these cells. Isolation of mRNA and Northen hybridization confirmed the colorimetric results.

Furthermore, in the experiments of the present invention, the toxicity of sodium cyanide upon the cells was evaluated. It was found that glioblastoma tumor cell lines (C6, L9 or U373 MG) as well as one glioblastoma explant from a human patient, are 10 to 500 times more resistant to the toxic effect of exogenous sodium cyanide than ΨCRIP. This finding could be related to the observation that malignant tumor cells (C6) have a low oxidative respiration and a decrease in the number of mitochondria making them quite resistant to metabolic poisons such as KCN (Lichtor and Dohrmann 1986; Maduh et al. 1991), a property that can be partly reversed *in vivo* by the low pH conditions maintained within the tumor. Neoplastic cells forming a tumor are exposed to a substantial and consistent decrease in extracellular pH from 7.4 to 6.6 in the case of glioblastoma, or lower for other type of tumors ( Hu et al. 1988; Gerweck and Seetharaman 1996) resulting in higher sensitivity (50% or greater) to the inhibitory effects of sodium cyanate. Nutrient deprivation due to poor vascularization within solid tumors is probably the major cause of this acidity, limited oxygen diffusion causes induction of anaerobic glycolysis and lactic acid acumulation.

*In vitro,* cultures are normally maintained closer to pH 7.4 than 6.6; however lowering the culture pH to 6.6 reduced the $IC_{50}$ of the cell lines tested. The lower interstitial pH in tumors than normal tissues would result in a greater and specific sensitivity to the toxic effect of cyanide on tumor cells. This is very convenient because it will selectively increase the specificity of the therapeutic action towards inner tumor cells rather than normal surrounding cells.

The type of euthanasic effect expected in the present system is rather different from the prototype bystander effect dependent on connexin expression and cell communication via gap junctions (Wygoda et al., 1997; Vrionis et al., 1997). Toxicity to the neighbours of a linamarase containing cell is due to cyanide release, and cyanide action does not require cell-to-cell contact or gap junctions.

In order to determine the bystander effect, experiments were performed in which linamarase producer cells were mixed in a fifty/fifty initial cell ratio with non producer cells. The results of these experiments indicate that the bystander effect in this system is very efficient, being proportional to the amount of cyanide released by the producer cells and the lethal dosage for cyanide in neighbouring cells.

Based on the encouraging *in vitro* data, the *in vivo* sensitivity of the system was evaluated using an intracerebral tumour model. Two types of experiments were performed: in the first type, C6 cells already transfected with *pLlinSp* and selected for puromycin resistance, were inoculated into the animal brain and allowed to form a tumour. The glioblastoma were subsequently treated with linamarine. In the second type of experiments, brain tumours were induced by ordinary C6 cells, and ΨCRIP *lin*-retroviral producing cells were inoculated into the growing tumour, 3 or 5 days later, tumours were expose to linamarin.

C6*lin* transfected cells were inoculated into the white matter of the right cerebral hemisphere of Wistar rats. A few weeks later an intracraneal tumour was clearly visible by magnetic resonance imaging (around 70 $mm^3$ volume size). After linamarin delivery (a total of 2.4 mg) throughout an 8 day period the magnetic resonance image taken three weeks after the treatment showed a total absence of the tumoral mass. One of the animals having a similar size tumour, was treated with only 1 mg of linamarin. The animal finished the treatment but finally died as a consequence of the tumour, indicating a lower limit dosage for the prodrug.

No apparent toxicity was observed by local administration of up to 9 mg of linamarin into normal rat brains in the same conditions as in the experimental animals.

In the second type of experiments, C6 cells were inoculated into the right hemisphere of Wistar rats and allowed to develop into tumours. Three of the largest size tumour animals, with approximate volume sizes of were selected for further inoculation with ΨCRIP *lin*-retroviral producing and local linamarine treatment. A total eradication of the tumour was achieved for the largest and the smallest glioblastomas.

Control animals with tumours about the same volume sizes, treated only with linamarin (6 mg) in the same conditions as described earlier, died shortly afterwards.

The cured animals were well for several months after the treatment, with no apparent neurological damage, and no recurrence. With the present invention, it was possible for the first time to eradicate a rat glioblastoma of the

dimensions described.

Toxicity to normal brain tissue was estimated by direct inoculation of ΨCRIP*lin* producer cells, followed by linamarin treatment. The obtained magnetic resonance images of the treated animals serve as a proof that the treatment itself does not produce appreciable damage to the brain of the animals.

Transduction rate of tumour cells by retroviral vectors *in vivo* is usually in the range of 1-10% of the tumour cell population. An efficient bystander (good Samaritan or euthanasic) effect has been hypothesized to explain the total remission of tumours in animal models by the *Hstk/ganciclovir* system. Transfer of phosphorylated ganciclovir between cells via gap junctions appears as one of the best documented theories to explain the mechanism (Wygoda et al., 1997; Vrionis et al., 1997). Some tumour cells, like rat glioblastoma C6 cells for example, have lost the capacity to synthesize some of the connexin proteins and therefore the benefit from this important effect will be severely limited. The linamarase/linamarin enzyme/prodrug combination does not require cell-to-cell contact for its bystander effect, since evidence of cyanide in the cell culture medium indicates that it can cross cell membranes. Furthermore, the bystander effect expected *in vivo* should be greater than *in vitro.* The volatile character of cyanide makes it rather difficult to estimate the killing effect for surrounding cells *in vitro* because the compound dissipates into the medium and air faster than to neighbouring cells. On the other hand, *in vivo,* within a tightly packaged tumour, the cells nearest to the producers will be the first and main target of the cyanide released. Cyanide released within a tumour would also be more effective against neighbouring cells by the low extracellular pH increased responsive effect (Hu et al., 1988; Gerweck and Seetharaman, 1996). Cells outside the tumour having extracellular pH values close to 7.4 would be rather resistant to the cyanide bystander effect.

In the experiments of the present invention considerations concerning possible toxic effects have been taken into account. One of the aspects that needed to be considered is related to the possibility of linamarin breakdown in other parts of the body by alternative enzymes or situations; and the other aspect concerns to the putative damage of healthy brain tissue surrounding the tumour.

Regarding the first issue, there exists one β-glucosidase in mammals which has broad specificity and an obscure physiological function which enables the organism to hydrolize toxic plant glycosides found in the diet. In general, the richest source of this cytosolic β-glucosidase in most vertebrates appears to be the liver and the small intestine except in the mouse and in the rat, where kidney is the richest source of the enzyme (Glew et al., 1993; Lima Górniak et al., 1993; Newmark et al., 1981). In experiments of the present invention it was confirmed that cyanogenic glycosides, such as linamarin, are not hydrolyzed by cultured mammalian cells, rat brain tumour or normal tissue and tumour or normal human brain tissue.

In addition, it has to be considered that gut microbial flora would be able to metabolize, to a certain extent, some cyanogenic glycosides such as amygdalin or linamarin, in the intestinal lumen by bacterial β-glucosidases, resulting in the release and absorption of hydrogen cyanide. Female hamsters given an oral dose of 108 mg of linamarin/kg body weight show signs of cyanide poisoning causing death in 18% of the animals (Frakes and Sharma, 1986; Hernandez et al., 1995). The toxicity is much lower in animals receiving similar doses intravenously and is practically absent when administered intraperitoneally (Glew et al., 1993; Lima Górniak et al., 1993; Newmark et al., 1981). Local administration of linamarin is as safe or probably safer than intraperitoneal delivery to avoid gastrointestinal interference and it is clear from the *in vitro* and *in vivo* tests of the present invention that the are well below dangerous concentrations of the prodrug. One alternative way for the oral administration of cyanogenic glucosides is to pre-treat the animals (or patients) with especially extended spectrum antibiotics to reduce gastrointestinal flora and be less susceptible to the compound toxicity (Newton et al., 1981; Padmaja, 1995; Halstrom and Moller, 1945).

In many tropical communities a potential health hazard is recognized by the consumption of leaves and roots of the cassava plant (*Manihot esculenta* Crantz). When mechanical damage of tissues of this plant occurs, the cyanogenic glucoside (linamarin) is exposed to the action of the endogenous plant linamarase and α-hydroxynitrile lyase and hydrocyanic acid is released. Cooking/boiling, drying, soaking and fermentation are the usual processing techniques to inactivate linamarase and breakdown linamarin before consumption, the occasional poisoning reported in humans is generally due to inadequately processed leaves or roots from cassava. Cyanide doses of 50 to 100 mg are reported to be lethal to adults (Newton et al., 1981; Padmaja, 1995; Halstrom and Moller, 1945). This would be equivalent to 3 mg /kg of sodium or potassium cyanide and to 500 to 1000 mg of linamarin. The dose of linamarin potentially needed to eradicate a human tumour of about 4 cm diameter, is below dangerous concentrations of the prodrug and would be administered over a week time period.

The second concern is related to the damage that cyanide release could cause in healthy parts of the brain. Cyanide can rapidly enter the plasmatic membrane and slowly accumulate in mitochondria and membrane elements of the neuronal cell, in a proportional way to the concentration available. An advantage for applying the present invention is the local extracellular lower pH created within tumours and the increased susceptibility of such condition to cyanide. Physiological extracellular pH values in surrounding normal cells will make them rather resistant to the cyanide bystander effect. In one of the *in vivo* experiments that have been performed, a large majority (if not all) of tumoral cell had incorporated the linamarase gene and therefore could process linamarin to glucose, acetone and cyanide (C6

transfected with *pLlinSp*). Nevertheless magnetic resonance images show a fibrous scar comparable to the one obtained previously with the *Hstk/ganciclovir* system (Culver et al., 1992; Ram et al., 1993; Izquierdo et al., 1995; Izquierdo et al., 1997) or to the C6 infected *in vivo* by retroviral-lin, indicating the absence of a devastating effect in the brain. All the surviving animals were healthy and well several months after the treatment, with no apparent signs of neurological damage and no recurrence of the tumour. Glioblastoma is a particularly nasty tumour because of the infiltrating characteristic of its cellular constituents, recurrence tumours are generally not farther than 2 cm from the primary site; it maybe difficult to eliminate infiltrated neoplastic cells without damaging some surrounding brain cells. In the past it has not been possible to cure rat glioblastomas larger than 150 $mm^3$ volume size using the *Hstk/ganciclovir* system (Izquierdo et al., 1997), implying that the new linamarase/linamarine approach is more efficient. When the *Hstk/ganciclovir* system was applied to a small number of patients by others and the inventors (Culver et al., 1994; Izquierdo et al., 1996; Roth and Christiano, 1997), a genuine tumour size reduction in about 30% of the treated patients was generally acknowledged, but no total disappearance of the residual tumour has been achieved despite several consecutive treatments. Survival time was increased in many cases but recurrence finally killed the patient. The method of the invention has yielded results in animal experiments that can be considered highly encouraging.

Brief description of the figures:

Fig. 1. Enzymatic action of linamarase. Schematic representation of linamarin breakdown by linamarase into glucose and acetone cyanohydrin and further spontaneous decomposition of this product releasing free cyanide and acetone

Fig. 2. Recombinant retroviral plasmid pL*linSp.*

Fig. 3. Sensitivity to linamarin as a percentage of cell survival.

A) ΨCRIP sensitivity to linamarin compared to one clone of retroviral *lin* infected ΨCRIP

B) C6 sensitivity to linamarin compared to one clone of retroviral *lin* transfected C6

C) FLYA13 sensitivity to linamarin compared with one clone retroviral *lin* infected FLYA13

Fig. 4 Gadolinium enhanced magnetic resonance views in sagittal ($a_1$,$b_1$) and axial ($a_2$, $b_2$) projections of a rat brain tumor, before ($a_1$, $a_2$) and after linamarin treatment ($b_1$, $b_2$)

Fig. 5 Gadolinium enhanced magnetic resonance views in axial ($a_1$,$b_1$) sagittal ($a_2$, $b_2$) and coronal ($a_3$, $b_3$) projections of a rat brain tumor, before ($a_1$, $a_2$ $a_3$ ) and after mouse retroviral producer cells ΨCRIP*lin* inoculation and linamarin treatment ($b_1$, $b_{2,,}$ $b_3$).

Fig. 6 Axial $T_2$( $a_1$, $b_1$) and sagittal $T_1$ ($a_2$, $b_2$) magnetic resonance views of two control animals

Fig. 7 Cyanide presence in tissue homogenates. (a) ΨCRIP*lin* (b) C6 (c) C6 *lin* (d) Normal rat brain (e) Normal human brain (f) Human glioblastoma.

In the experiments of the invention, the following materials and methods were used

a) Construction of a recombinant plasmid encoding linamarase
The plasmid pCas 5 (Hughes et al., 1992) was opened with the restriction enzyme SacII and filled with Klenow, then cut with Sal I to generate the fragment blunt-Sal I. The fragment was inserted in pBabe Puro (Morgenstern and Land, 1990), opened via Sna B1/Sal I restriction enzymes (Sna B1 gives blunt end, so the insert was oriented by the Sal enzyme). The recombinant (Fig. 2) is called *pLlinSp* and can be selected by its resistance to puromycin (1-2,5 μg/ml puromycin) under the promoter of SV40; *linamarase* is under the LTR promoter. The prokaryotic moiety allowed for growing the recombinant plasmid in bacteria prior to transduction of mammalian cells.

b) Cell lines and culture
Cells: C6 (ATCC CLL 107) and L9 (rat glioblastoma; Weizsaecker et al., 1981), MGU 373 (U-373 MG; ATCC HTB-17; human glioblastoma), NIH-3T3 (ATCC CRL6331, ΨCRE (ecotropic packaging line; Danos and Mulligan, 1988) ,ΨCRIP (amphotropic packaging line; Danos and Mulligan, 1988), Ag9181 (human fibroblast), and FLYA13 (Cosset et al., 1995) were grown in Dulbecco modified Eagles medium (DMEM). Some were supplemented with 10% heat inactivated fetal calf serum: C6, L9, MGU 373, Hs683, Ag9181) and the rest in calf serum. Cells were cultured at

37°C, 6% $CO_2$ and 97% relative humidity. The pH of DMEM was artificially lowered to pH 6.6 in some experiments by decreasing the $NaHCO_3$ concentration from 3.7 g/L to 0.7 g/L.

c) Transfections and infections

The retroviral plasmid *pLlinSp* was introduced to ecotropic ΨCRE packaging cells using the AVET technique for DNA delivery (Cotten, et al., 1992; Wagner, et al., 1992; von Rüden, et al., 1995), and clones were selected by puromycin (48h later). The same plasmid was introduced to ecotropic ΨCRE packaging cells. Transfections were essentially performed as described previously (von Rüden, et al., 1995). 4 μg pLlinSl in 150 μl HBS were used to transfect 200000 cells during 4 hours at 37°C.

Thereafter, the medium containing the transfection complex was replaced by fresh culture medium, containing 10 μmol dexamethason. 48 h later, for selection, 2 mg/ml puromycin was added to the cells. Two weeks later the colonies were isolated by using cloning rings.

Viral supernatants from lawns, collected 48 h after transfection, were used to infect amphotropic ΨCRIP packaging cells (ΨCRIP *lin* ). After selection with puromycin (1,5 μg/ml) colonies were isolated using cloning rings. Clonal lines were assayed for vector titer, using NIH 3T3 cells. Best titers were 3-6 x$10^5$ c.f.u./ml.

Cell lines shown in table 1 were infected with ΨCRIP*lin* supernatants (U373MG, Hs683, Ag9181, Sk-N-MC and FLYA13) or ΨCRE*lin* supernatants (C6 and 3T3), to obtain *lin* $^+$ cell lines. After selection with puromycin, colonies were isolated and linamarin sensitivity was estimated.

d) Labeling of cells

Cells were labeled essentially as described previously (Tamura et al., 1997). Two milligrams of Dil (1,1'-dioctedecyl-3,3,3',3'-tetramethyl-indocarbocyanine perchlorate) were dissolved in 1ml of ethanol, and 50 μl of the solution was added to 5ml of cells in a 6-cm culture dish. After one hour incubation at 37° C, the cells were trypsinized, washed twice with PBS, and resuspended in DMEM at a concentration of 5 x $10^4$ cells/μl.

e) Linamarin sensitivity and cyanide toxicity assays in culture

Cells were seeded in a flat sided tube (provided with screw cap to prevent HCN release), at a density of $10^5$ cells/tube. Following 24 h of incubation, increasing linamarin concentrations were added. Two days later, the percentage of surviving cells was determined to evaluate drug toxicity. Cells were incubated for 2.5 h with the mitochondrial substrate MTT (3-[4,5-dimethylthiazo-2-yl]-2,5-diphenyltetrazolium bromide, 200 μg/ml) which can be transformed in formazan only by living cells. Then the medium was removed and 3 ml of DMSO (dimethylsulfoxide) were added to dissolve formazan. Samples were measured spectrophotometrically at 540 nm after 10 min.

Cyanide toxicity was assayed as described previously except by the addition of increasing amounts of NaCN to the cultures.

f) Measurement of linamarase activity and cyanide presence

Linamarase activity and cyanide presence were determined by the Lambert method (Lambert et al., 1975) with the following modifications: 1ml of the succinimide/N-chlorosuccinimide reagent was added to the flat tubes, containing the cells with differents amounts of linamarin (48 h later), cultured in 1 ml of DMEM without phenol red. Then 1 ml of the barbituric acid/pyridine reagent was added, mixed and the absorbtion at 592 nm was read after 10 min (calibration curves were made with NaCN).

g) Tissue homogenates

The tissues were minced and homogenized in five volumes of a medium containing sucrose (0.25 M), ethylenediamine tetraacetate (1 mM) and Tris HCl buffer (0.01 M, pH 7.2) supplemented with the protease inhibitor phenylmethylsulfonyl fluoride (PMSF) 1 mM (all procedures were carried out to 1-4°C). The homogenate was centrifuged (600xg, 10 min).The supernatant fraction was stored at -70°C (Glew, R. et al. 1976). The protein content was measured by BCA Protein Assay.

The experiment in Fig. 7 was carried out at 37°C, 4 hours of incubation with 1 mM linamarin, 0.2 mg protein, pH 7.4, in a total volume of 1 ml. Cyanide presence was determined by the Lambert method (Lambert et al., 1975).

h) Wistar rat brain inoculation of C6 lin transfected cells and linamarin administration.

The procedures applied were essentially those described previously (Izquierdo et al., 1995, Izquierdo et al., 1997). Wistar male rats weighing 250-300 grams were anesthetized with a mixture of ketamine (50 mg/ml), valium (5 mg/ml) and atropin (1mg/ml) in a 5:4:1 ratio, by vol. at a dose of 0.3 ml/100 g of body weight before placing them in a stereotaxic apparatus. C6 glioma cells or the same cells transfected with the *pLlinSp* plasmid and selected by *puromycin* resistance were injected at a concentration of $10^5$ cells/μl in complete PBS (with calcium and magnesium) supplemented with 0.1% glucose. With the aid of the manipulating arm of the stereotaxic apparatus a total

of 10 µl were introduced, over a 5 minutes interval, into the fronto-parietal lobe of the right cerebral hemisphere (4 mm to the right from Bregma and 4.5 mm deep from the skull) using a 10 µl Hamilton syringe connected to a 26 gauge needle; the needle was kept in place 3 min. before and after injection. ΨCRIP *lin* producer cells were inoculated at the same stereotaxic coordinates as the C6 cells except in a very large tumour in which an additional tumour site was also reached, cells were delivered at different heights along the tumour. All rats received tetracycline into the drinking water (approximately 75 mg/kg) and dexamethasone (1 mg/500 ml) for three days before and one week after surgery. The linamarin treatment was started after magnetic resonance visualization of the tumour and size estimation. The prodrug was administered by a brain infusion cannula placed at the site of tumour inoculation and fixed to the skull using dental cement; a small stainless steel screw acts as anchor to secure the cannula and the dental cement. The brain infusion cannula was then connected subcutaneously (s.c.) to an osmotic pump (alzet® model 2001) which delivers 1 µl/h for 7 or 8 days. The osmotic pump was filled with the appropriate amount of linamarin in PBS .

j) Magnetic resonance imaging
Sagittal and coronal views of the skull were made with a Cpflex small coil arround the animal. T1 sagittal and axial proyection images were obtained using spin eco, with TR 650, TE 17.0/1 and adquisition 2, slide 3 mm. Field of view 50x100. Matrix 256x512 high resolution sequence post enhancement with gadolinium (0,2 ml/Kg) with magnetization transfer MT.

k) Estimation of tumour volume
Volumes of tumours were estimated as follows. If [0,L] is a parametrization of the tumour interval in the *sagittal* section and C (*x*), $0 \leq x \leq L$ denotes the area of the corresponding *coronal* section, then we have the exact formula for the volume of the tumour

$$V=\int_0^L C(x)dx,$$

which yields the approximation

$$V_{app}=\frac{L}{N}\sum_{j=1}^{N} C\left(j\frac{L}{N}\right) \cdot$$

In practice, however, the volume was needed to be estimated with the knowledge of only one section of each type. These tumours are ussually adapted to the rat's head geometry: they tend to be rather symmetric (elliptical) in the coronal sections, while they have long and irregular sagittal shapes.
Given two "well taken" sections, coordinates were introduced:

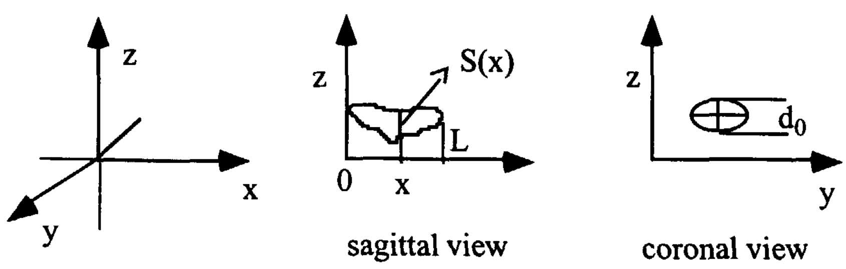


A plausible model for the tumour is given by the volume generated placing sections homothetical to $C_0$ at each position *x* with a ratio $S(x)/d_0$.

Therefore the area ratio is $[S(x)/d_0]^2$

$$V \cong \frac{C_0}{d_0^{\,2}} \frac{L}{N} \sum_{j=1}^{N} \left(S\left(j\frac{L}{N}\right)\right)^2$$

Where:

$C_0$ = area of the given *coronal* section.
$d_0$ = diameter of that *coronal* section in the z (sagittal) direction.
S(x) = length of the *sagittal* section in the z direction at position x.
N was to chosen taking into account the shape of the *sagittal* picture.

It is possible to obtain similar formulas changing sagittal by axial. However, taking into consideration the standard geometries of these tumours, the axial section seems to be less significative than the sagittal one. In general, it was expected to have an estimate from below using the *axial-coronal* approach.

Example 1

Construction of recombinant retrovirus carrying the linamarase gene

A therapeutic retrovirus was engineered by inserting the linamarase gene (*cas 5*; Hughes et al., 1992) into the Moloney murine leukemia based vector pBabe*puro* (Morgenstern und Land, 1990). The resulting recombinant retroviral plasmid carries the *cas 5* gene expressed under the control of the 5' LTR (long terminal repeat) promoter and the selection gene *pac* (puromycin N-acetyl transferase) under the SV40 promoter (Fig. 2). The estimated size of the retroviral plasmid was 6.9 kilobases (kb) and was designated *pLlinSp* (plasmid, LTR, *linamarase*, SV40 promoter, *puromycin N-acetyl transferase*). The recombinant *pLlinSpL* can be selected by its resistance to puromycin (1-2,5 μg/ml puromycin) under the promoter of SV40; *linamarase* is under the LTR promoter.

Example 2

Assessment of linamarin sensitivity of retroviral infected cells and toxicity of sodium cyanide for the cells

a) Linamarin sensitivity as a percentage of cell survival.

i) ΨCRIP sensitivity to linamarin was compared to one clone of retroviral *lin* infected ΨCRIP, at increasing linamarin concentrations and two pH values. The initial number of cells was $10^5$ in all cases. Following 24 hours incubation, increasing linamarin concentrations were added. Two days later, the percentage of surviving cells was determined to evaluate drug toxicity. Clonal lines were assayed for vector titer, using NIH 3T3 cells. Best titers were 3-6 x$10^5$ c.f.u./ml.

The results of the experiment are shown in Fig. 3a. Some clones showed a marked toxicity to the presence of linamarin, with estimated concentrations that resulted in 50% cytotoxicity ($IC_{50}$), of 7-60 μg/ml.

ii) C6 sensitivity to linamarin was compared to one clone of retroviral *lin* infected C6, at increasing linamarin concentrations and two pH values. The initial number of cells was $10^5$ in both cases and the estimated $IC_{50}$ for this C*6lin* clone is 345 μg/ml at pH 7.4 and 202 μg/ml at pH 6.6. The results of the experiment are shown in Fig. 3b.

iii) FLYA13 sensitivity to linamarin was compared with one clone retroviral *lin* infected FLYA13 at increasing linamarin concentrations and two pH values. The estimated $IC_{50}$ for this FLYA13*lin* clone is 195 μg/ml at pH 7.4 and 77 pg/ml at pH 6.6. The results of the experiment are shown in Fig. 3c.

The sensitivity of other cell lines, whose $IC_{50}$ was determined in the same manner, is shown in table 1.

b) Cyanide toxicity

Next, the toxicity of sodium cyanide upon the cells was evaluated. The chemical compound itself is an order of magnitude (ten times) more toxic than linamarin in both ΨCRIP and C6. This result was interpreted as showing that only 10% of the linamarin applied to the medium ends up as cyanide. It was also found that glioblastoma tumour cell

lines (C6, L9 or U373MG) as well as one glioblastoma explant from a human patient, are 10 to 500 times more resistant to the toxic effect of exogenous sodium cyanide than ΨCRIP (and therefore 3T3, from which ΨCRIP have derived), a property that can be partly related to the pH conditions maintained *in vitro* and *in vivo.*

Example 3

Infectivity and cyanide bystander effect

In order to determine the bystander effect in this system, experiments were performed in which linamarase producer cells were mixed in a fifty/fifty initial cell ratio with non producer cells. One of the mixtures was the packaging cell line ΨCRIP infected with the retrovirus carrying *lin* (ΨCRIP*lin*) plus normal C6 cells labeled red with Dil (1,1'-dioctedecyl3,3,3',3'-tetramethyl-indocarbocyanine perchlorate (Tamura, et al., 1997). In the presence of 2.5 mg/ml of linamarin, both types of cells, (ΨCRIP*lin* and C6 labeled) died; while 86% of C6 alone, will survive at linamarin concentrations of 10 mg/ml. Lower concentrations of linamarin (100 μg/ml) affect only partially the viability of either cell type in this mixture, despite the fact that ΨCRIP alone would be very sensitive to this concentration (Fig. 3a). These results were interpreted as showing that C6 cells, which are more resistant to cyanide than ΨCRIP, are trapping sufficient cyanide to lower the free concentration in the medium. Mixtures of retroviral carrying *lin* infected ΨCRIP with either ΨCRIP or 3T3 labeled in red (ΨCRIP*lin* /ΨCRIP red or ΨCRIP*lin* /3T3 red) were devastated at the lower concentration of linamarin (100 μg/ml) due to the greater sensitivity of red labeled cells to cyanide released by producer cells. The results indicate that the bystander effect in this system is very efficient, being proportional to the amount of cyanide released by the producer cells and the lethal dosage for cyanide in neighbouring cells.

Example 4

Curability of rat glioblastoma in vivo

Based on the encouraging *in vitro* data, the *in vivo* sensitivity of the system was evaluated by using an intracerebral tumour model. Two types of experiments were performed: in the first type, C6 cells already transfected with *pLlinSp* and selected for puromycin resistance, were inoculated into the animal brain and allowed to form a tumour. The glioblastoma would be subsequently treated with linamarin. In the second type of experiments, brain tumours were induced by C6 ordinary cells and CRIP *lin*-retroviral producing cells were inoculated into the growing tumour, 3 or 5 days later, tumours were expose to linamarin.

A total of $10^6$ C6*lin* transfected cells were inoculated, in the first type of experiments, into the white matter of the right cerebral hemisphere (4 mm to the right of Bregma and 4.5 mm deep from the skull) of three Wistar rats. A few weeks later (2 to 4), an intracraneal tumour was clearly visible by magnetic resonance imaging (around 70 $mm^3$ volume size) (Fig. 4 $a_1$,$a_2$). A day later, linamarin was administered by a direct brain-tumour infusion cannula connected by a catheter tube to a osmotic mini-pump. A total of 2.4 mg of linamarin were delivered throughout an 8 day period. Release was at a constant rate of 1μl per hour (12.5 μg of linamarin/μl).

Fig. 4 shows the gadolinium enhanced magnetic resonance views in sagittal ($a_1$,$b_1$) and axial ($a_2$, $b_2$) projections of a rat brain tumor, before ($a_1$, $a_2$) and after linamarin treatment ($b_1$, $b_2$). A brain tumor (70 $mm^3$ volume size, $a_1$ $a_2$ arrow) of glioblastoma-C6*lin* transfected cells, has been eradicated completely ($b_1$ $b_2$ arrow) after one week treatment with constant local release of 12,5 μg of linamarin per hour. The magnetic resonance image taken three weeks after the treatment (Fig. 4 $b_1$,$b_2$), shows a total absence of the tumoural mass and in its place a residual hypointense image at the site of injection, interpreted as a fibrous scar or as a small rest of haematoma with internal haemosiderin. One of the animals having a similar size tumour, was treated with only 1 mg of linamarin. The animal finished the treatment but finally died as a consequence of the tumour, indicating a lower limit dosage for the prodrug.

In the second type of experiments, $10^6$ C6 cells were inoculated into the right hemisphere of 10 more Wistar rats and allowed to develop into tumours. Three of the largest size tumour animals (volume estimation shown in materials and methods), with approximate volume sizes of 400 $mm^3$, 600 $mm^3$ and 1000 $mm^3$ (Fig.5 $a_1$,$a_2$, $a_3$), were selected for further inoculation with ΨCRIP *lin*-retroviral producing cells (7x$10^6$ cells for the two smallest and $10^7$ cells for the largest) and linamarine local treatment. The murine retroviral producer cells (ΨCRIP*lin*) had a titer of 5x$10^5$ c.f.u. (colony forming units) and were inoculated in a single place (the same used for C6 delivery) in all but the biggest size tumour in which an additional small craniotomy was made at a distal side within the tumour, in order to increase the potential access of retrovirus to all neoplastic cells. A few days later (three for the largest tumour and 5 for the others), 6 mg of linamarin in 200 μl were administered at a constant rate of 1 μl per hour (osmotic pump delivery). A total eradication of the tumour was achieved for the largest (Fig. 5 $b_1$,$b_2$, $b_3$ ) and the smallest glioblastomas; the medium size tumour animal, on the other hand, finished also the treatment but died a month later. Examination of the dead animal brain showed an haemorrhage within the tumour remains being probably the cause of death. Fig. 5 shows the gadolinium enhanced magnetic resonance views in axial ($a_1$,$b_1$) sagittal ($a_2$, $b_2$) and coronal ($a_3$, $b_3$) projections of a rat brain, before ($a_1$, $a_2$ $a_3$ ) and after mouse retroviral producer cells ΨCRIP*lin* inoculation and linamarin treatment ($b_1$, $b_2$, $b_3$). As can be seen, a very large brain tumour (approximately 1000 $mm^3$ volume size, $a_1$ $a_2$ $a_3$ arrows) of rat glioblastoma C6 cells, has been eradicated completely as shown in the three magnetic resonances ($b_1$ $b_2$ and $b_3$ arrows). These images show the appearance of the living brain after murine therapeutic retroviral producer cells inoculation into the tumour (ΨCRIP*lin*) and one week treatment, three days later, with constant local release of 30 μg of linamarin per hour.

Two control animals with tumours of 220 and 140 $mm^3$ volume sizes, that have been treated only with the prodrug (6 mg) in the same conditions as described earlier, died shortly afterwards.

The cured animals were well, with no apparent neurological damage, and no recurrence.

Some of the treated animals showed no apparent neurological damage for a period up to six months, but developed a recurrence later. This was only seen in very large tumors (over 500 $mm^3$ in size) in approximately 50% of the cases. In all recurrent cases the tumoral mass had crossed the interhemispheric fissure and reached the ventricles. At this point tumoral cells could be distributed via cerebrospinal fluid to parts of the brain quite distant from the original inoculum. Indeed some of the recurrent tumors were multiple and very far from the original site.

Fig. 6 shows the axial $T_2$( $a_1$, $b_1$) and sagittal $T_1$ ($a_2$, $b_2$) magnetic resonance views of two control animals treated in the right hemisphere with $5x10^6$ mouse *lin*- retroviral producer cells and then with 4 (a) and 6 mg of linamarin (b). The left hemisphere image can be considered as a control of untreated brain tissue.

Toxicity to normal brain tissue was estimated by direct inoculation of ΨCRIP*lin* producer cells, followed by linamarin treatment (2.5, 4 or 6 mg) in the absence of tumor cells. The magnetic resonance of the treated animals did not show any large damage, but a small scar similar to the one normally present in brains after the tumor has been eradicated (Fig. 6). Two types of relaxation time (in Tesla, T) images were analyzed: $T_1$ and $T_2$ in order to visualize lesions other than tumoral. In general, $T_1$-weighted images primarily provide anatomic information, $T_2$-weighted images are more sensitive to early pathologic changes in most tissues. No lesion related to neoplasm, edema, ischemia, infection, demyelination or trauma; was seen animals only exhibited small MRI abnormalities and no gross lesions. These results may be interpreted as a proof that the treatment itself does not produce appreciable damage to the brain of the animals.

Example 5

Toxicity experiments

β-glucosidase and linamarase activities were estimated in several tissues from rats and humans. It was expected to obtain β-glucosidase but no linamarase activity in most tissues assayed . The tissues tested from the rat were: normal brain, glioblastoma C6, liver, kidney and gut. Normal brain and cells from a glioblastoma explant were tested from humans. The tissues were minced and homogenized in five volumes of: 0.25 M sucrose, 1 mM ethylenediamine and 0.01 MTris-HCl pH 7.2, and 1 mM of phenylmethylsulfonyl fluoride as protease inhibitor; at 1 - 4°C. The homogenate was centrifuged (10 min. 600xg) and supernatants stored at -70°C. The protein content was measured by BCA protein assay (Pierce).

The enzyme determinations of glucosidase and linamarase activity were carried out at 37°C. Glucosidase activity was measured as release of p-nitrophenol (yellow, absorbance at 400 nm) from p-nitrophenyl-β-D-glucoside.

Linamarase activity was estimated as cyanide release measured by the Lambert method (Lambert et al., 1975). In this assay flat side tubes with screw-cap to avoid cyanide release were used. 0.1, 0.2, 0.5 and 1 mg of tissue protein were estimated. The amounts of linamarin were 1 mM, 5 mM, 10 mM and 20 mM. Reaction was measured at two pH values pH 6 and the physiological pH 7.4. Positive control were transfected C6lin cells and transfected YCRIP-lin cells. All human tissues gave negative results for linamarase activity. In rats only the kidney gave low but detectable levels of cyanide production.

Fig. 7 shows the presence of cyanide in tissue homogenates. (a) ΨCRIP*lin* (b) C6 (c) C6 *lin* (d) Normal rat brain (e) Normal human brain (f) Human glioblastoma.

Table 1

| Sensitivity of different cell lines to linamarin | | | |
|---|---|---|---|
| Cell line | % of survival cells with 1 mg/ml Linamarin | $IC_{50}$ µg/ml Linamarin *lin* + clones | |
| | | Best clone | Mean value[b] |
| C6 Rat glioma | 100[a] | 345 | 407 |
| U-373 MG Human Glioblastoma | 80 | 145 | 236 |
| Hs 683 Human Glioma | 80 | 230 | 288 |
| Sk-N-MC Human Neuroblastoma | 80 | 183 | 215 |
| FLYA13 Human Amphotropic Packaging Cells | 88[a] | 195 | 382 |
| ΨCRIP Murine Amphotropic Packaging Cells | 85 | 7 | 32 |
| NIH 3T3 Murine Fibroblasts | 90[a] | N.D. | N.D. |
| Ag 9181 Human Fibroblasts | 89[a] | N.D. | N.D. |

N. D.: not determined.
[a] In these cell lines the concentration of linamarin was increased to 5 mg/ml without a substantial drop in survival values. In the other cell lines was not determined.
[b] Mean value determined at least with 4 different clones. Clones with $IC_{50}$ higher than 500 µg/ml were not considered.

References

Abaza, MS. et al., 1997, J. Cancer Res., 88 (11) 1094-1099
Andreansky, S.S., et al., 1996, Proc Natl Acad Sci USA, Vol. 93, Oct., 11313-11318
Bilbao, G., et al., 1997, FASEB J, Vol. 11, 624-634
Bonnekoh, B., et al., 1995, J Invest Dermatol, 104, 313-317
Braas, G., et al., 1996, Bioseparation, 6, 211-228
Chen, S.H., et al., 1994, Proc Natl Acad Sci USA, 91, 3054-3057
Cirielli, C., et al., 1997, J Neuro-Oncology, 31, 217-223
Cosset, F-L., et al., 1995, J. of Virology, 69, 7430-7436.
Gotten, M., et al., 1992, Proc.Natl. Acad. Sci. USA, 89, 6094-6098
Culver, K. W., et al., 1992, Science, 256, 1550-1552
Culver, K., et al., 1994, Human gene therapy, 5, 343-379
Danos, O., and Mulligan, 1988, Proc. Nat. Acad. Sci. USA, 85, 6460-6464.
Deonarain, 1994, Gene Therapy, 1, 149-151
Descamps, V., et al., 1996, J Mol Med, 74, 183-189
Fieldes, M.A., and Gerhardt, K.E., 1994, Electrophoresis, 15, 654-661
Felgner, P.L., et al., 1987, Proc Natl Acad Sci USA, 84, 7413-7417
Felgner, J.H., et al., 1994, J Biol Chem, 269, 2550-2561
Frakes, R.A., and Sharma, R.P., 1986, Fd Ghem. Toxic.24, 417-420
Frehner, M., and Conn, E.E., 1987, Plant Physiol., 84, 1296-1300
Gerweck, L. E., and Seetharaman, K., 1996, Cancer Research, 56, 1194-1198
Glew, R. H., et al., 1993, from *β-glucosidases: biochemistry and molecular* biology. ed.A. Zsen, Am. Chem. Soc. Symp., series 533
Gmelin, R., et al., 1973, Phytochemistry, 12(2), 457-461
Guenzburg, W.H. and Salmons, B., 1995, Molecular Med Today, Reviews, 410-417
Guenzburg, W.H., et al., 1996, Cytokines and Molec Therapy, Vol. 2, 177-184
Halstrom, F., and Moller, K.D., 1945, Acta Pharmacol. Toxicol., 1, 18
Hernandez, T., et al., 1995, Natural Toxins, 3, 114-117
Hu, J. J., et al., 1988, Biochem. Pharmacol., 37, 2259-2266

Hug, P. and Sleight, R.G., 1991, Biochimica at Biophysica Acta, 1097, 1-17
Hughes, M.A., and Dunn, M.A., 1982, Plant Molecular Biology, 1, 169-181
Hughes, M. A., et al., 1992, Archives of Biochemistry and biophysics, 295, 273-279
Itoh-Nashida, T., et al., 1987, J. Biochem., 101, 847-854
Izquierdo, M., et al., 1995, Gene Therapy, 2, 66-69
Izquierdo, M., et al., 1996, Gene Therapy, 3, 491-495
Izquierdo, M., et al. 1997, Acta Neurochirurgica, suppl. 68, 111-117
Jensen, S.R., and Nielsen, B.J., 1973, Acta Chem. Scand., 27(7), 2661-2
Kakes, P., 1985, Planta, 166, 156-160
Kovesdi, I., et al., 1997, Curr Opin Biotechnol, Oct, 8(5), 583-589
Kramm, C.M., et al., 1995, Brain Pathology, 5, 345-381
Lambert, J. L., et al., 1975, Analytical Chemistry, 47, 916-918
Latchman, D.S., 1994, Molecular Biotechnol., Vol. 2, 179-195
Lichtor T., and Dohrmann, G. J., 1986, Neurosurgery, 19, 896-899
Lima Górniak, S., et al., 1993, J. of Ethnopharmacology, 38, 85-88
Maduh, E. U., et al., 1991, Journal of applied toxicology, 11, 97-101
Mao, C.H., and Anderson, L., 1967, Phytochemistry, 6(4), 473-483
Mkpong, O. E., et al., 1990, Plant Physiol., 93, 176-181
Morgenstern, J. P., and Land, H., 1990, Nuc. Acids Res, 18, 3587-3596
Nartey, F., 1968, Phytochemistry, 7(8), 1307-1312
Newmark, J., et al., 1981, Proc. Natl. Acad. Sci. USA, 78, 6513
Newton, G. W., et al., 1981, West J. Medicine., 134, 97-103
Nishi, T., et al., 1996, Cancer Res, 56, 1050-1055
Oxtoby, E., et al., 1991, Plant Molecular Biology, 17, 209-219
Padmaja, G., 1995, Crit. Rev. in Food and Nut., 35, 299
Peltékian, E., et al., 1997, J Neuroscience Methods, 71, 77-84
Ram, Z., et al., 1993, Cancer research, 53, 83-88
Roth, J.A., and Cristiano, R.J., 1997, Journal of the National Cancer Institute, 89, 21
Runnebaum, I.B., 1997, Anticancer Res, 17, 2887-2890
Rüden von, T., et al., 1995, BioTechniques, 18, 484-489
Sambrook, J., et al., 1989, Molecular Cloning: A laboratory Manual, $2^{nd}$ ed. Cold Spring Harbor Laboratory Press, Cold Spring Harbor, NY
San, H., et al., 1993, Hum Gene Ther, 4, 781-788
Selmar, D. et al., 1987 a), Plant Physiol., 83, 557-563.
Selmar, D., et al., 1987 b), Analyt. Biochemistry, 166, 208-211
Stevens, D.L., and Strobel, G.A., 1968, J. Bacteriol., 95(3), 1094-102
Tamura, M., et al., 1997, Human Gene Therapy, 8, 381-391
Vile, R.G. and Russell, S.J., 1995, British Med Bulletin, Vol. 51, No. 1, 12-30
Vrionis, F. D., et al., 1997, Gene Therapy, 4, 577-585
Wagner, E., et al., 1992, Proc. Natl. Acad. Sci. USA, 89, 6099-6103
Weizsaecker et al., 1981, J. Neurology, 224, 183-191
Wygoda, M. R., et al., 1997, Cancer research, 57, 1699-1703
Yoshida, J., and Mizzuno, M., 1994, J Neuroonc, 19, 269-274
Yung, W.K.A, 1994, Curr Opinion Neurol, 7, 501-505

## Claims

1. Use of a DNA molecule encoding a glycosidase together with a non-toxic cyanogenic glycoside substrate which upon degradation by the glycosidase yields hydrogen cyanide, for the preparation of a medicament for separate administration of the two interacting components in the treatment of brain tumours in animals or humans.

2. Use according to claim 1, wherein the glycosidase is a glucosidase.

3. Use according to claim 2, wherein the glucosidase is a β-glucosidase.

4. Use according to claim 3, wherein the β-glucosidase is linamarase.

5. Use according to any of claims 1 to 4, wherein the DNA is carried by a viral vector.

6. Use according to claim 5, wherein the vector is a retroviral vector.

7. Use according to claim 6 wherein the titer is $\geq 10^6$ colony forming units.

8. Use according to claim 6, wherein the medicament contains packaging cells that have been infected with retroviral vectors.

9. Use according to claim 8, wherein the medicament contains $\geq 10^9$ cells.

10. Use according to claim 5, wherein the viral vector is an adenoviral vector.

11. Use according to claim 5, wherein the viral vector is a Herpes Simplex vector.

12. Use according to claim 1, wherein the DNA molecule is carried by a plasmid contained in a liposome.

13. Use according to claim 1, wherein the substrate is a cyanogenic monosaccharide.

14. Use according to claim 2 and claim 13, wherein the monosaccharide is glucose.

15. Use according to claim 14, wherein the substrate is linamarin.

16. Kit for the treatment of brain tumours, wherein a first container contains the preparation with the DNA encoding the glycosidase, and wherein a second container contains the cyanogenic substrate.

17. Kit according to claim 16, wherein the glycosidase is linamarase and the substrate is linamarin.

18. Kit according to claim 16 or 17, wherein the preparation with the DNA contains retroviral vectors.

19. Kit according to claim 18, wherein the preparation with the DNA contains producer cells that have been infected with retroviral vectors.

Fig. 1

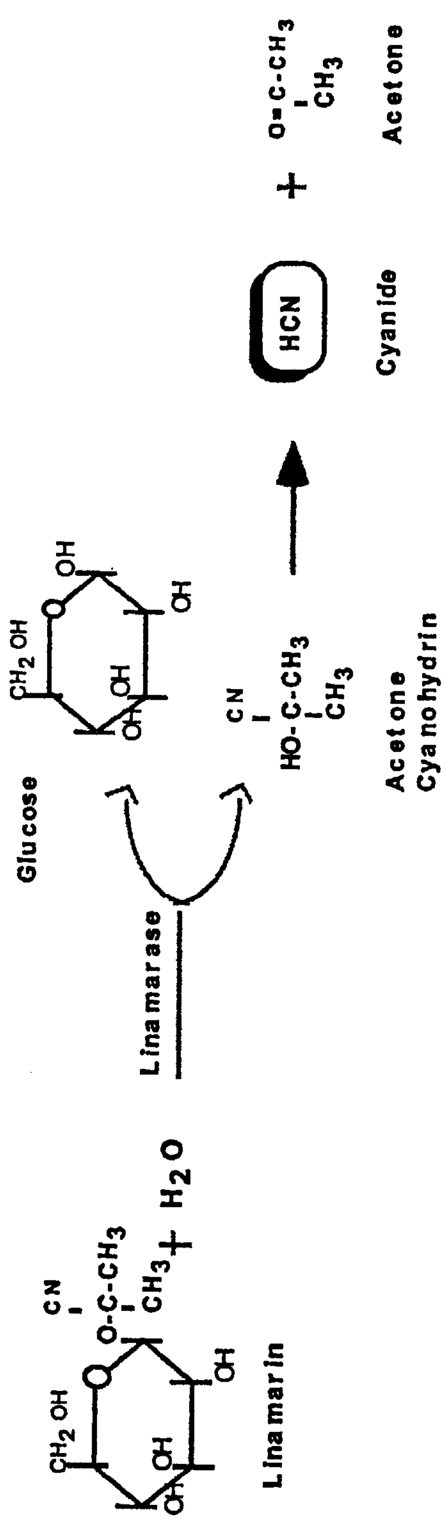


Linamarin

Glucose

Acetone Cyanohydrin

Cyanide

Acetone

Fig. 2

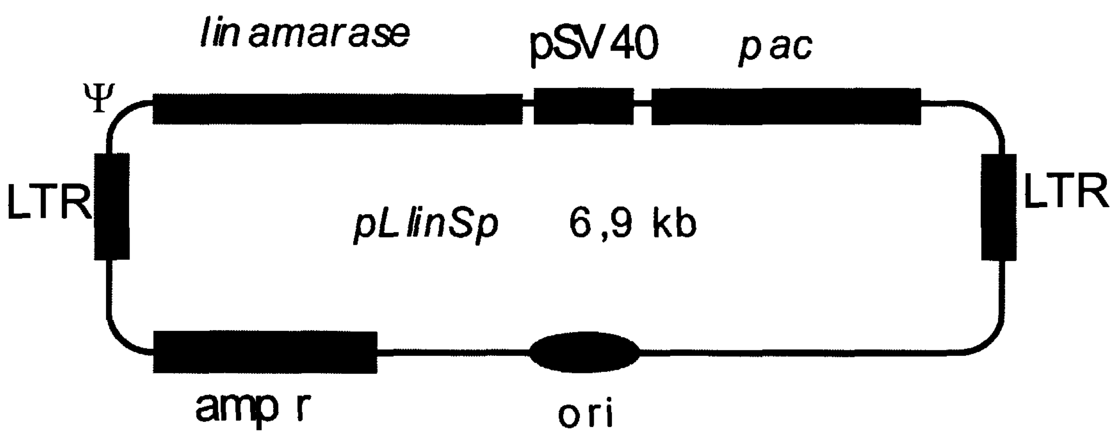

Fig. 3

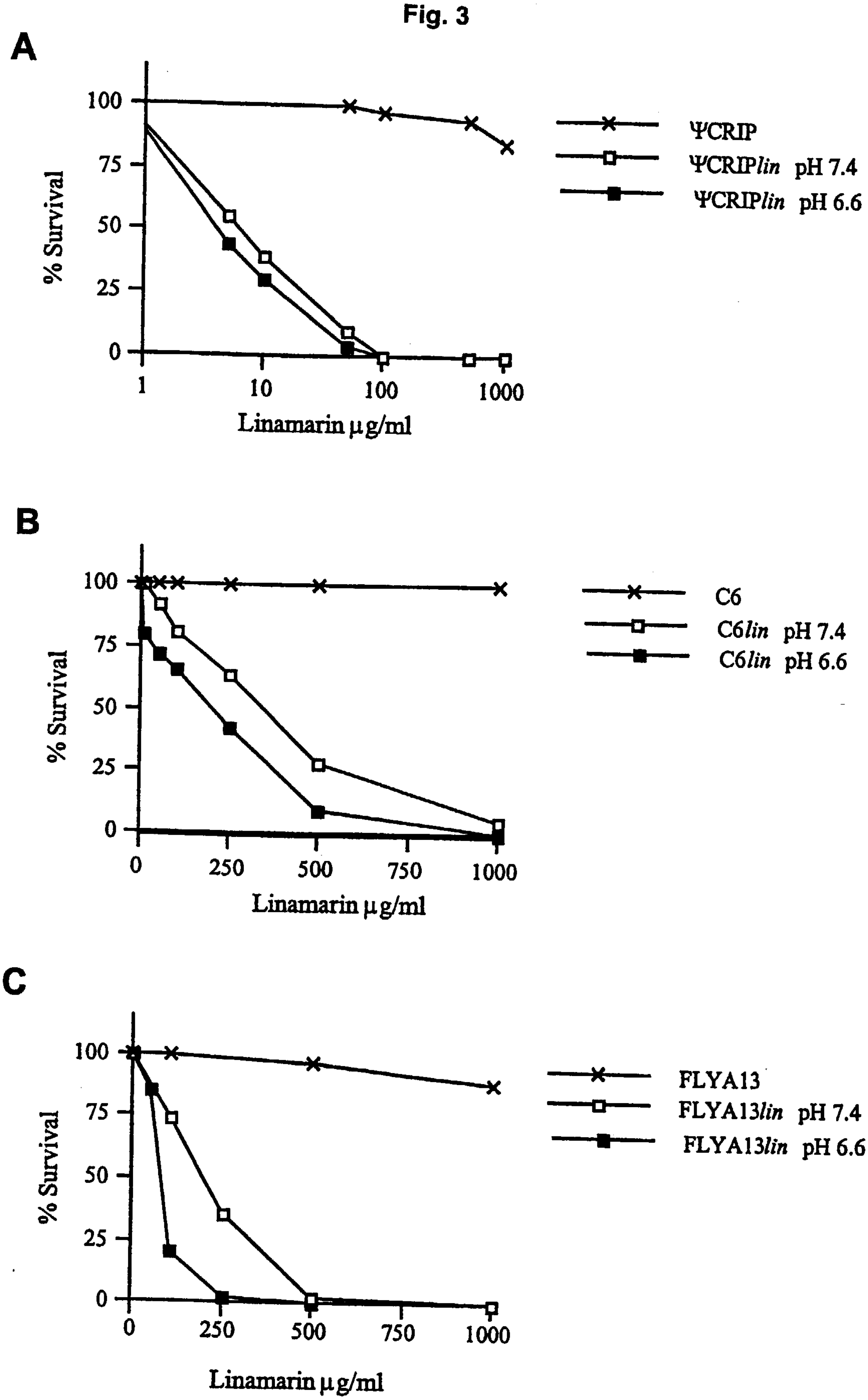
A
100
75
50
25
0
% Survival
1
10
100
1000
Linamarin µg/ml
ΨCRIP
ΨCRIP*lin* pH 7.4
ΨCRIP*lin* pH 6.6
B
100
75
50
25
0
% Survival
0
250
500
750
1000
Linamarin µg/ml
C6
C6*lin* pH 7.4
C6*lin* pH 6.6
C
100
75
50
25
0
% Survival
0
250
500
750
1000
Linamarin µg/ml
FLYA13
FLYA13*lin* pH 7.4
FLYA13*lin* pH 6.6

Fig. 4

a1

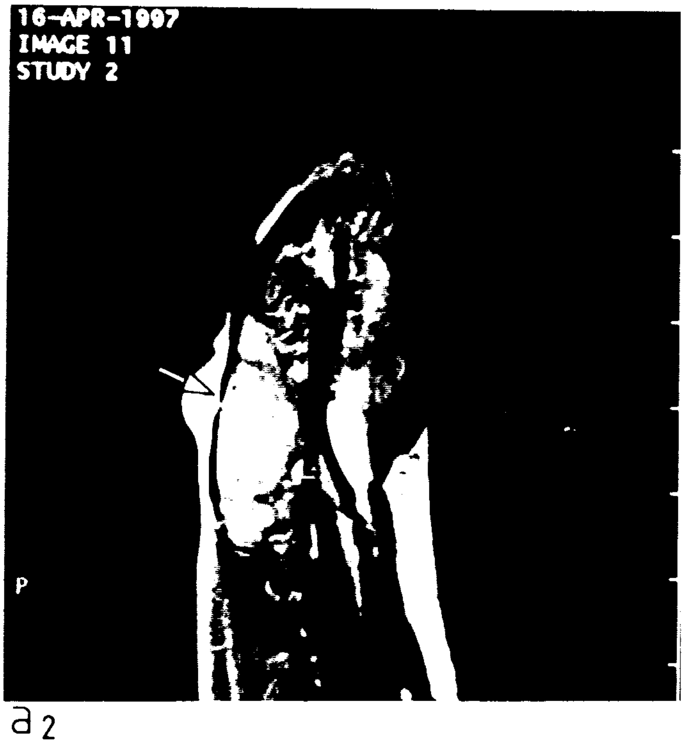


b1

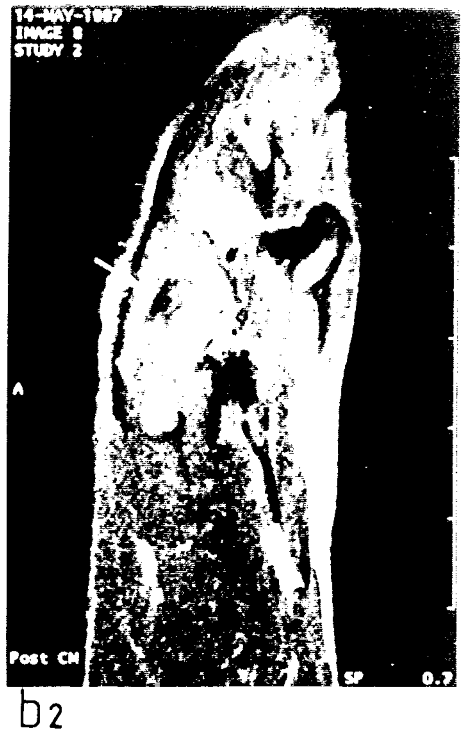


a2

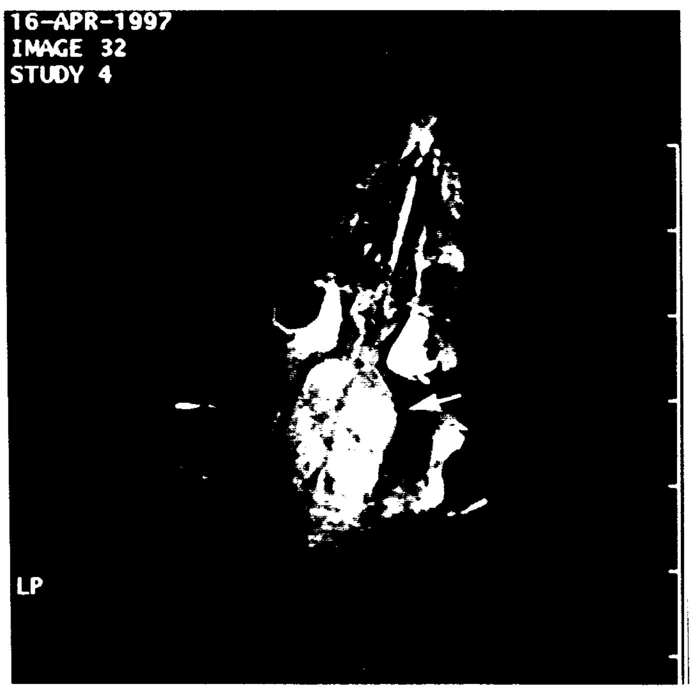


b2

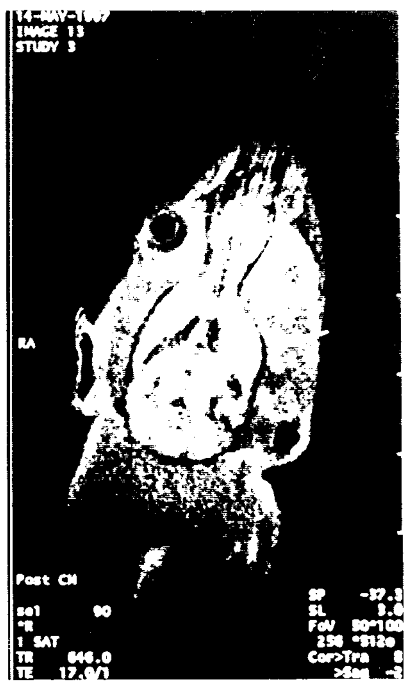

Fig. 5

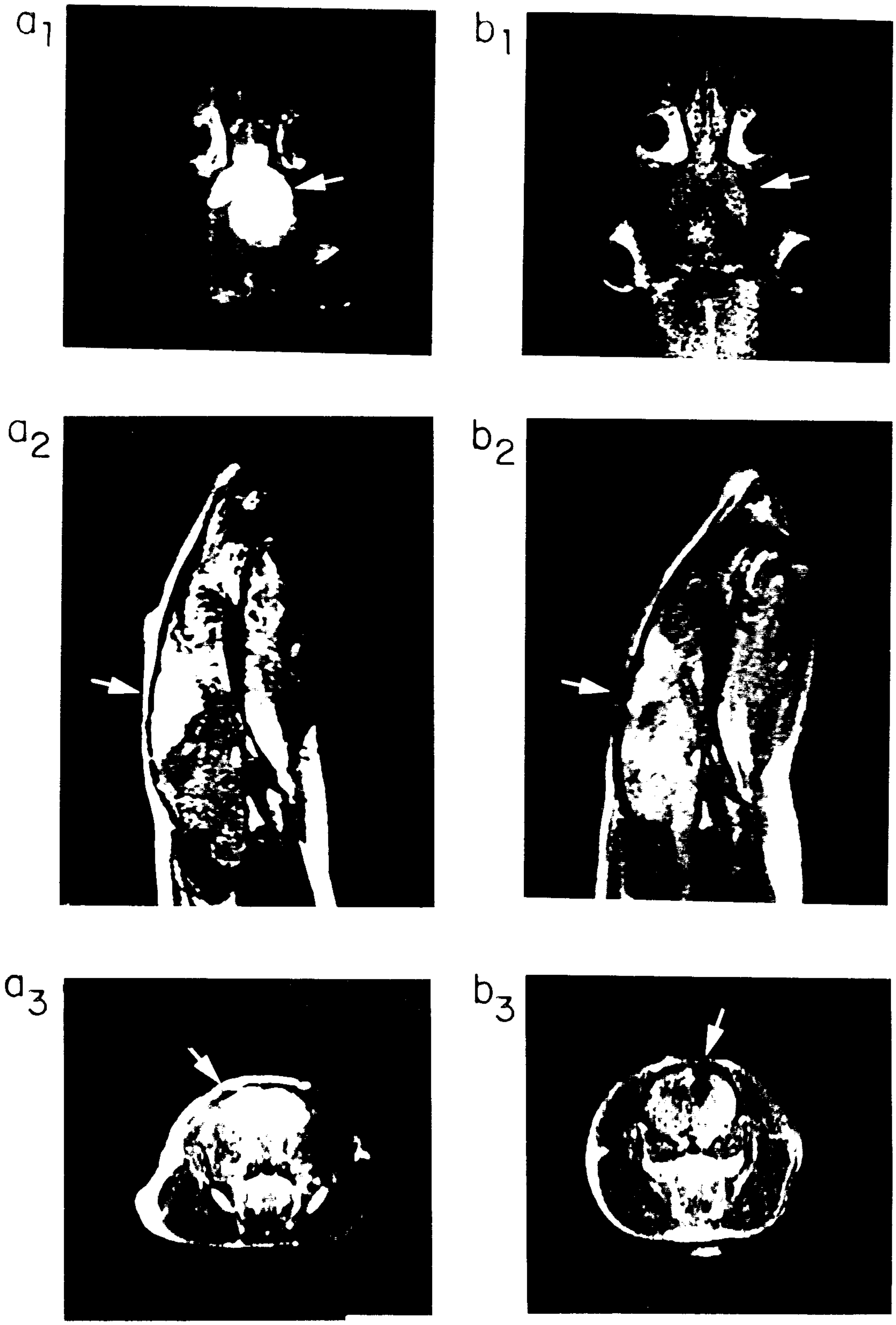

Fig. 6

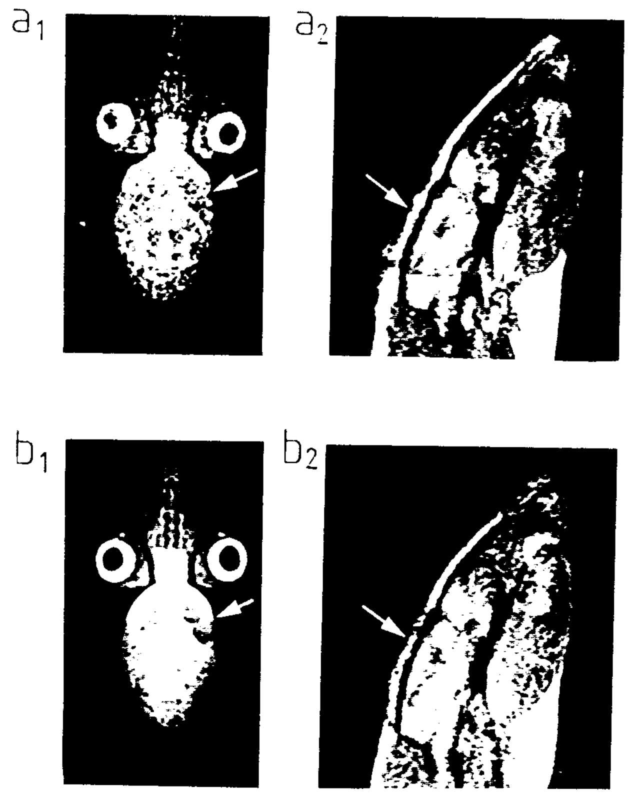

Fig. 7

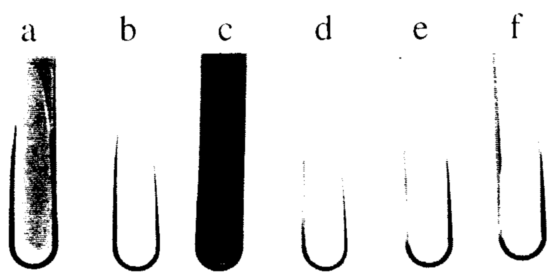

European Patent Office

# EUROPEAN SEARCH REPORT

Application Number
EP 98 10 4756

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | WO 94 28127 A (IMPERIAL CANCER RESEARCH TECHNOLOGY ) 8 December 1994<br>* page 9, line 10 - page 10, line 5 *<br>* page 12, line 23 - page 15, line 15 *<br>* page 17, line 13 - page 19, line 15 *<br>* page 17, line 3 - line 10 * | 1 | C12N9/42<br>A61K48/00 |
| A | | 2,3,5,6,8,12,13,16 | |
| Y | WO 97 44065 A (CYTOTHERAPEUTICS INC) 27 November 1997<br>* page 3, line 25 - line 34 *<br>* page 13, line 29 - page 19, line 11 *<br>* page 21, line 25 - page 24, line 25 * | 1 | |
| A | | 5,6,8,9,16 | |
| A | DEONARAIN M P ET AL: "GENETIC DELIVERY OF ENZYMES FOR CANCER THERAPY"<br>GENE THERAPY,<br>vol. 2, no. 4, June 1995, pages 235-244, XP002036307<br>BASINGSTOKE GB<br>* page 236 - page 237; table 1 *<br>* page 240, column 1, paragraph 3 * | 1-6,13-17 | TECHNICAL FIELDS SEARCHED (Int.Cl.6)<br>C12N<br>A61K |
| A | WO 91 11201 A (IMPERIAL CANCER RESEARCH TECHNOLOGY) 8 August 1991<br>* page 7, last paragraph - page 8 * | 1-4,13-17 | |

-/--

The present search report has been drawn up for all claims

| Place of search | Date of completion of the search | Examiner |
|---|---|---|
| BERLIN | 14 September 1998 | De Kok, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 98 10 4756

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | OLDFIELD E H ET AL: "GENE THERAPY FOR THE TREATMENT OF BRAIN TUMORS USING INTRA-TUMORAL TRANSDUCTION WITH THE THYMIDINE KINASE GENE AND INTRAVENOUS GANCICLOVIR" HUMAN GENE THERAPY, vol. 4, no. 1, February 1993, pages 39-69, XP002059072 NEW YORK US * the whole document * --- | 1,5,6,8, 16 | |
| A | WO 93 04167 A (UNIVERSITY OF CALIFORNIA) 4 March 1993 * the whole document, especially page 13, line 34 - line 37 --- | 1,5,10, 11 | |
| A | EP 0 795 325 A (INSTITUTE OF APPLIED BIOCHEMISTRY ) 17 September 1997 * the whole document * --- | 12 | |
| D,A | HUGHES M A ET AL: "A molecular and biochemical analysis of the structure of the cyanogenic beta glucosidase linamarase from cassava manihot-esculenta cranz" ARCHIVES OF BIOCHEMISTRY AND BIOPHYSICS, vol. 295, no. 2, 1992, pages 273-279, XP002075032 NEW YORK US * the whole document * ----- | 1,4,16, 17 | TECHNICAL FIELDS SEARCHED (Int.Cl.6) |

The present search report has been drawn up for all claims

| Place of search | Date of completion of the search | Examiner |
|---|---|---|
| BERLIN | 14 September 1998 | De Kok, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)